# EUROPÄISCHE PATENTSCHRIFT

(11) **EP 1 359 410 B1**
(45) Veröffentlichungstag und Bekanntmachung des Hinweises auf die Patenterteilung: **02.03.2005**
(21) Anmeldenummer: 03006612.0
(22) Anmeldetag: 25.03.2003
(51) Int. Cl.: G01N 27/22, G01N 27/02, G01N 22/04

(54) **Vorrichtung zur Bestimmung der Feuchtigkeit eines Untergrundes**
Device for determining the humidity of a substrate
Dispositif pour la détermination de l'humidité d'un substrat

(30) Priorität: 30.04.2002 DE 20206903 U
(43) Veröffentlichungstag der Anmeldung: 05.11.2003
(73) Patentinhaber: imko Intelligente Micromodule Köhler GmbH, 76275 Ettlingen (DE)
(72) Erfinder: Köhler, Kurt, 76275 Ettlingen (DE)
(74) Vertreter: Dimmerling, Heinz, Dipl.-Ing.

(56) Entgegenhaltungen:
- EP-A- 0 478 815
- EP-A- 0 924 511
- EP-A- 0 990 627
- DE-A- 10 159 340
- DE-A- 19 502 803

## Beschreibung

Die Erfindung betrifft eine Vorrichtung nach dem Oberbegriff des Anspruchs 1, zur Bestimmung der Feuchtigkeit eines Untergrundes, bei welcher der Untergrund das Dielektrikum einer Meßanordnung bildet, und welche Vorrichtung eine Auswerteelektronik zur Bestimmung der Dielektrizitätskonstante des Untergrundes aufweist, mit einem Speicher zur Speicherung von Kalibrationsdaten, mittels der eine jeweils ermittelte Dielektrizitätskonstante in einen Feuchtigkeitswert umgewandelt wird.

Eine derartige Vorrichtung ist beispielsweise aus der EP 0 478 815 B1 bekannt und wird von der Patentanmelderin mit großem Erfolg vermarktet. Bei der bekannten Vorrichtung bildet der Untergrund das Dielektrikum einer Meßleitung, in welche an einem Ende mittels eines Signalgebers ein pulsförmiges Signal eingespeist wird. Das am anderen Ende der Meßleitung reflektierte Signal wird an dem einen Ende von einem Empfänger empfangen. Mittels einer Meßeinrichtung wird die Zeit, die zwischen dem Beginn des Einspeisens des Signals und dem Eintreffen des reflektierten Signals verstreicht, gemessen. Hierdurch läßt sich die Dielektrizitätskonstante des Untergrundes sehr genau bestimmen. Da die Dielektrizitätskonstante des Untergrundes von der Feuchtigkeit abhängt, läßt sich mittels der Kalibrationsdaten eine jeweils mittelte Dielektrizitätskonstante in einen entsprechenden Feuchtigkeitswert umwandeln.

Neben der vorstehend beschriebenen Vorrichtung ist jedoch auf dem Markt noch eine Vielzahl anderer Vorrichtungen bekannt, mittels welcher ebenfalls über die Messung der Dielektrizitätskonstante die Feuchtigkeit eines Untergrunds bestimmbar ist. Wenngleich sich die Verfahren zur Messung der Dielektrizitätskonstanten auch unterscheiden, so enthalten die bekannten Vorrichtungen jedoch alle einen Speicher, in dem jeweiligen Untergründen zugeordnete Kalibrationsdaten gespeichert sind, mittels welcher eine jeweils gemessene Dielektrizitätskonstante in einen entsprechenden Feuchtigkeitswert umgewandelt wird.

Zwar werden die bekannten Vorrichtungen in vielfältiger Weise eingesetzt, jedoch ist es nicht bekannt, diese zur Bestimmung der Feuchtigkeit eines Zement oder Kalziumsulfat aufweisenden Untergrundes, insbesondere zur Bestimmung der Feuchtigkeit eines Zement- oder Kalziumsulfat-Estrichs zu verwenden. Dies dürfte an der bei der Bestimmung der Feuchtigkeit eines Zement oder Kalziumsulfat aufweisenden Untergrundes vorhandenen Problematik liegen.

Die Bestimmung der Feuchtigkeit eines Estrichs ist von besonderer Bedeutung, da die weitere Bearbeitung eines aus Estrich bestehenden Bodens von der Feuchtigkeit des Estrichs abhängt. So darf die Feuchtigkeit des Estrichs insbesondere dann einen bestimmten Wert nicht überschreiten, wenn der Estrich mit einem Bodenbelag versehen werden soll. Erst wenn die Feuchtigkeit des Estrichs einen bestimmten Wert, die sogenannte Verlegereife, erreicht hat, kann der Estrich mit einem Bodenbelag versehen werden. Die Verlegereife ist jedoch bei einem Zement-Estrich anders als bei einem Kalziumsulfat-Estrich. So kann die Verlegereife bei einem Zement-Estrich beispielsweise bei einem Feuchtigkeitswert von 3 Prozent erreicht werden, wohingegen sie bei einem Kalziumsulfat-Estrich erst bei einem Feuchtigkeitswert von 0,5 Prozent erreicht wird.

Zur Bestimmung der Feuchtigkeit eines Estrichs sind verschiedene Verfahren bekannt. Die genaueste bekannte Methode ist die sogenannte Gravimetrische Methode. Bei ihr wird dem Estrich eine Probe entnommen, welche dann während einer vorbestimmten Zeit getrocknet wird. Aufgrund der sich ergebenden Gewichtsdifferenz läßt sich die Feuchte des Estrichs bestimmen.

Die Anwendung der gravimetrischen Methode ist jedoch bei Zement-Estrich und Kalziumsulfat-Estrich unterschiedlich. So wird Zement-Estrich beispielsweise bei einer Temperatur von 105 Grad Celsius getrocknet, wohingegen Kalziumsulfat-Estrich bei einer Temperatur von 40 Grad Celsius getrocknet wird. Für die Anwendung der gravimetrischen Methode ist es daher erforderlich, die Art des Estrichs zu kennen. Problematisch beziehungsweise nahezu nicht mehr durchführbar ist die gravimetrische Methode dann, wenn der Estrich aus einem Gemisch von Zement-Estrich und Kalziumsulfat-Estrich besteht. Des weiteren hat die gravimetrische Methode den Nachteil, daß sie regelmäßig nur im Labor durchgeführt werden kann.

Zur Bestimmung der Feuchtigkeit von Estrich ist weiterhin die sogenannte Kalziumkarbid-Methode bekannt. Bei ihr wird dem Estrich ebenfalls eine Probe entnommen, welche dann in ein Behältnis gegeben wird, in dem sie mit Kalziumkarbid vermengt wird. Aufgrund der entstehenden chemischen Reaktion steigt der Druck im Behältnis an, wobei der Druckanstieg um so größer ist, je feuchter der Estrich ist. Somit läßt sich aufgrund des Druckanstiegs die Feuchte des Estrichs bestimmen.

Die bei der Kalziumkarbid-Methode gewonnenen Ergebnisse entsprechen in etwa den mit der Gravimetrischen Methode gewonnenen Ergebnissen. Jedoch weisen die Ergebnisse bei Zement-Estrich einen Offset in etwa von 1,2 Prozent auf. Das heißt, würde man bei einem Zement-Estrich mit der gravimetrischen Methode eine Feuchtigkeit von 6 Prozent ermitteln, würde man bei Anwendung der Kalziumkarbid-Methode einen Wert von 4,8 Prozent erhalten. Daraus ergibt sich, daß ein Zement-Estrich, welcher bei einer mit der gravimetrischen Methode ermittelten Feuchtigkeit von 3 Prozent die Verlegereife erreicht hat, die Verlegereife bei der Kalziumkarbid-Methode bei 1,8 Prozent erreicht.

Bei Feuchtigkeitswerten eines Kalziumsulfat-Estrichs ist dieser Offset nicht vorhanden. Das heißt, die mittels der Kalziumkarbid-Methode gewonnenen Feuchtigkeitswerte eines Kalziumsulfat-Estrichs entsprechen den Feuchtigkeitswerten, welche mit der gravimetrischen Methode gewonnen worden sind. Somit hat ein Kalziumsulfat-Estrich, der bei einer mittels der gravimetrischen Methode gewonnenen Feuchtigkeit von 0,5 Prozent seine Verlegereife erreicht hat, ebenfalls bei einer mittels der Kalziumkarbid-Methode ermittelten Feuchtigkeit von 0,5 Prozent seine Verlegereife reicht.

Da die Feuchtigkeit bei der Verlegereife bei Zement-Estrich und Kalziumsulfat-Estrich unterschiedlich hoch ist, ist es somit auch bei der Kalziumkarbid-Methode von besonderer Bedeutung zu wissen, ob es sich um einen Zement-Estrich oder einen Kalziumsulfat-Estrich handelt. Sofem es sich bei dem verwendeten Estrich um ein Gemisch aus Zement-Estrich und Kalziumsulfat-Estrich handeln sollte, läßt sich mittels der Kalziumkarbid-Methode die Verlegereife nahezu nicht mehr bestimmen.

Es ist Aufgabe der Erfindung, eine eingangs genannte Vorrichtung derart auszubilden, daß mit ihr die Feuchtigkeit eines Zement und/oder Kalziumsulfat aufweisenden Untergrundes auf einfache Weise bestimmbar ist.

Die Lösung dieser Aufgabe ergibt sich aus den Merkmalen des kennzeichnenden Teils des Anspruchs 1.

Gemäß der Erfindung ist eine Vorrichtung zur Bestimmung der Feuchtigkeit eines Untergrundes, bei welcher der Untergrund das Dielektrikum einer Meßanordnung bildet, und welche Vorrichtung eine Auswerteelektronik zur Bestimmung der Dielektrizitätskonstanten des Untergrundes aufweist, mit einem Speicher zur Speicherung von Kalibrationsdaten, mittels der eine jeweils ermittelte Dielektrizitätskonstante in einen Feuchtigkeitswert umgewandelt wird, dadurch gekennzeichnet, daß zur Bestimmung der Feuchtigkeit eines Zement aufweisenden Untergrundes dieselben Kalibrationsdaten verwendet werden, wie zur Bestimmung der Feuchtigkeit des Kalziumsulfat aufweisenden Untergrundes.

Dadurch, daß zur Bestimmung der Feuchtigkeit eines Zement aufweisenden Untergrundes dieselben Kalibrationsdaten verwendet werden, wie zur Bestimmung der Feuchtigkeit eines Kalziumsulfat aufweisenden Untergrundes, ist es in vorteilhafter Weise nicht mehr erforderlich, zwischen einem Zement aufweisenden Untergrund und einen Kalziumsulfat aufweisenden Untergrund zu unterscheiden. Es hat sich überraschend gezeigt, daß die Abhängigkeit und der Verlauf der Dielektrizitätskonstante von der Feuchtigkeit bei einem Zement aufweisenden Untergrund in etwa genauso groß ist, wie die Abhängigkeit und der Verlauf der Dielektrizitätskonstante bei einem Kaiziumsulfat aufweisenden Untergrund. insbesondere hat sich herausgestellt, daß der Wert der Dielektrizitätskonstante eines Zement aufweisenden Untergrundes bei einer mit der gravimetrischen Methode ermittelten Feuchtigkeit von drei Prozent in etwa genauso groß ist wie die Dielektrizitätskonstante eines Kalziumsulfat aufweisenden Untergrundes bei einer mit der gravimetrischen Methode ermittelten Feuchtigkeit von 0,5 Prozent.

Somit lassen sich zur Bestimmung der Feuchtigkeit eines Zement aufweisenden Untergrundes in vorteilhafter Weise dieselben Kalibrationsdaten verwenden, wie zur Bestimmung der Feuchtigkeit eines Kalziumsulfat aufweisenden Untergrundes. Aufgrund einer bestimmten gemessenen Dielektrizitätskonstante läßt sich beispielsweise feststellen, daß, sofern es sich bei dem Untergrund um einen Zement-Estrich handelt, dieser eine Feuchtigkeit von 3 Prozent aufweist, oder, sofern es sich bei dem Untergrund um ein Kalziumsulfat-Estrich handelt, dieser eine Feuchtigkeit von 0,5 Prozent aufweist.

Da die mittels der Messung der Dielektrizitätskonstante erhaltenen Feuchtigkeitswerte nahezu denselben Verlauf haben, unabhängig davon, ob es sich bei dem gemessenen Untergrund um einen Zement-Estrich oder einen Kalziumsulfat-Estrich handelt, ist es daher zum einen nicht mehr erforderlich zu wissen, ob es sich bei dem zu überprüfenden Untergrund um einen Zement-Estrich oder um einen Kalziumsulfat-Estrich handelt. Zum anderen lassen sich mittels der erfindungsgemäßen Vorrichtung Untergründe überprüfen, welche Zement und Kalziumsulfat aufweisen. Besonders vorteilhaft ist es hierbei auch, daß über das Verhältnis der Bestandteile des Gemischs nichts bekannt sein muß.

Weitere Einzelheiten, Merkmale und Vorteile der vorliegenden Erfindung ergeben sich aus der nachfolgenden Beschreibung eines besonderen Ausführungsbeispiels unter Bezugnahme auf die Zeichnung.

Es zeigt
- Figur 1: eine schematische Anordnung einer erfindungsgemäßen Vorrichtung und
- Figur 2: ein Diagramm, in dem der Feuchtigkeitsverlauf eines Zement-Estrichs und eines Kalziumsulfat-Estrichs, ermittelt mit der gravimetrischen Methode, gegenüber dem Feuchtigkeitsverlauf desselben Zement-Estrichs und Kalziumsulfat-Estrichs, ermittelt mit der erfindungsgemäßen Vorrichtung, dargestellt ist.

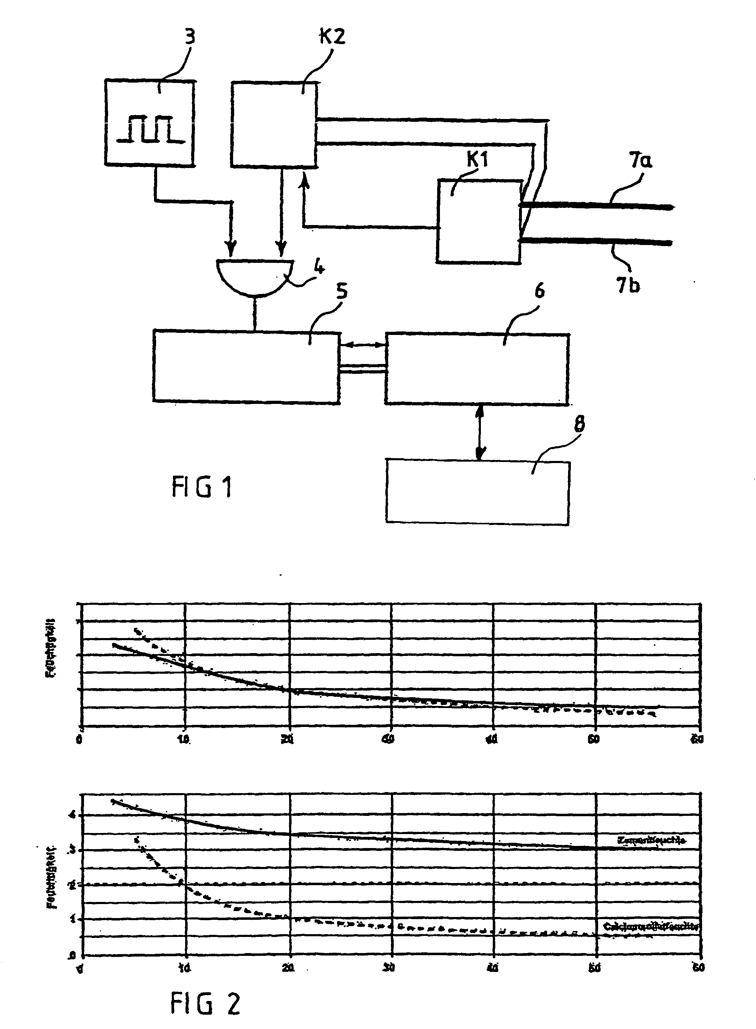

Wie Figur 1 entnommen werden kann, wird auf einer am Ende offenen Meßleitung, bestehend aus zwei parallelen Meßstäben 7a, 7b wird ein Spannungsanstieg mit möglichst steiler Signalflanke durch eine Signalquelle K1 erzeugt, die mit vemachlässigbarer Länge der Zuleitung an die Meßleitung angeschlossen ist. Der Innenwiderstand der Signalquelle K1 entspricht dem Wellenwiderstand der offenen Leitung 7a, 7b, soweit dieser mit einfachen Mitteln für die Mitte des Meßbereichs nachbildbar ist. Auf diese Weise entstehen an der Verbindung zwischen dem Ausgang der Signalquelle K1 und der Meßleitung 7a, 7b bei geeignetem Meßbereich wenig störende Reflexionen. Am offenen Ende der Meßleitung 7a, 7b tritt volle Reflexion auf. Das Echo erreichte den Anfang der Leitung nach einer Zeit, die abhängig von den durch die Feuchte des Dielektrikums beeinflußten physikalischen Daten der Leitung 7a, 7b ist. Durch einen Schwellwertschalter K2, der die Spannung am elektrischen Anschluß der Meßstäbe 7a, 7b mit vorgegebenen Werten vergleicht, wird ein Torsignal für einen Zähler 5 erzeugt, der nur in der Zeit zwischen Beginn der Erregung und dem Eintreffen des Echos Impulse eines freilaufenden Oszillators 3 zählt. Der Zähler 5 ist seinerseits mit einer Auswerte- und Anzeigeeinrichtung 6 verbunden. Der Beginn der Toröffnung kann vorteilhaft auch durch die Signalquelle K1 verzögert eingeleitet wenden, so daß eine Öffnung erste erfolgt, nachdem die Reflexion am Anschluß der Meßstäbe vorüber ist.

Durch Veränderung der Materialfeuchte ändert sich die Dielektrizitätskonstante und damit der Kapazitätsbelag des Mediums, das die Stäbe der Meßleitung 7a, 7b umgibt. Nachdem die geometrischen Daten der Meßleitung 7a, 7b konstant sind, folgt daraus ein bekannter Zusammenhang zwischen Materialfeuchte und gemessener Laufzeit des Echos. Dieser kann durch eine Gleichung oder eine Eichkurve gegeben sein, welche als Kalibrationsdaten in einem Speicher 8 hinterlegt sind.

Der Speicher 8 kann Daten für eine erste Kalibrierstufe aufweisen, welche beispielsweise steife und dichte Estriche betreffen, und Daten für eine zweite Kalibrierstufe aufweisen, welche beispielsweise plastische und geringer dichte Estriche betreffen. Als Estrich kommt beispielsweise ein Zement-Estrich, ein Kalziumsulfat-Estrich oder eine Estrich Mixtur in Frage. Des weiteren kann der Speicher 8 Daten für weitere Kalibrierstufen enthalten, welche Estriche betreffen, welche mit den Kalibrierstufen eins und zwei nicht vermessen werden können.

Die Daten der in der Auswerte- und Anzeigeeinrichtung 6 ermittelten Dielektrizitätskonstante werden an den Speicher 8 angelegt, der diese aufgrund der in ihm enthaltenen Kalibrationsdaten in die Zementfeuchte, Anhydritfeuchte und volumetrische Feuchte umwandelt und an die Auswerte- und Anzeigeeinrichtung 6 zurückgibt. Die Auswerte- und Anzeigeeinrichtung 6 kann gleichzeitig die Zementfeuchte und die Anhydritfeuchte ausgeben. Des weiteren kann gleichzeitig noch die volumetrische Feuchte ausgegeben werden.

Wie Figur 2 entnommen werden kann, ist der im oberen Teil der Figur 2 dargestellte mittels der erfindungsgemäßen Vorrichtung ermittelte Feuchtigkeitsverlauf eines Zement aufweisenden Estrichs (durchgehende Linie) und eines Kalziumsulfat aufweisenden Estrichs (gestrichelte Linie) nahezu gleich. Der im unteren Teil von Figur 2 dargestellte mittels der gravimetrischen Methode ermittelte Feuchtigkeitsverlauf des Zement-Estrichs (durchgehende Linie) erreicht nach etwa fünfzig Tagen die Verlegereife von 3 Prozent, wohingegen der Kalziumsulfat-Estrich (gestrichelte Linie) nach fünfzig Tagen etwa die Verlegereife von 0,5 Prozent erreicht hat. Für die Ermittlung der Feuchtigkeit ist es somit bei einer Messung mittels der erfindungsgemäßen Vorrichtung nicht von Bedeutung, ob der Estrich ein Zement-Estrich oder ein Kalziumsulfat-Estrich ist.

## Patentansprüche

1. Vorrichtung zur Bestimmung der Feuchtigkeit eines Untergrundes, bei welcher der Untergrund das Dielektrikum einer Meßanordnung (7a, 7b) bildet, und welche Vorrichtung eine Auswerteelektronik (6) zur Bestimmung der Dielektrizitätskonstante des Untergrundes aufweist, mit einem Speicher (8) zur Speicherung von Kalibrationsdaten, mittels der eine jeweils ermittelte Dielektrizitätskonstante in einen Feuchtigkeitswert umgewandelt wird,
**dadurch gekennzeichnet,**
**daß** der Speicher (8) zur Bestimmung der Feuchtigkeit eines Zement aufweisenden Untergrundes dieselben Kalibrationsdaten hat, wie zur Bestimmung der Feuchtigkeit eines Kalziumsulfat aufweisenden Untergrundes.

## Claims

1. A device for determining the moisture of a substratum, in which the substratum forms the dielectric of a measuring arrangement (7a, 7b), this device having an evaluation electronics unit (6) for determining the relative permittivity of the substratum, with a memory (8) for storing calibration data, by means of which a respectively determined relative permittivity is converted into a moisture value,
**characterised in that**
the memory (8) for determining the moisture of a substratum that contains cement has the same calibration data as for determining the moisture of a substratum that contains calcium sulphate.

## Revendications

1. Dispositif pour déterminer l'humidité d'un substrat, dans lequel le substrat forme le diélectrique d'un dispositif de mesure (7a, 7b), le dispositif comportant une unité électronique d'évaluation (6) destinée à déterminer la constante diélectrique du substrat, comportant une mémoire (8) qui permet d'enregistrer des données d'étalonnage au moyen desquelles une constante diélectrique respectivement déterminée est convertie en une valeur d'humidité,
**caractérisé en ce que** la mémoire (8), pour déterminer l'humidité d'un substrat présentant du ciment, a les mêmes données d'étalonnage que pour déterminer l'humidité d'un substrat présentant du sulfate de calcium.
